# EUROPÄISCHE PATENTSCHRIFT

(11) **EP 2 361 075 B1**
(45) Veröffentlichungstag und Bekanntmachung des Hinweises auf die Patenterteilung: **17.04.2013**
(21) Anmeldenummer: 10727352.6
(22) Anmeldetag: 19.05.2010
(51) Int. Cl.: A61H 35/02

(54) **AUGENSPÜLVORRICHTUNG**
EYE WASH DEVICE
DISPOSITIF DE LAVAGE POUR LES YEUX

(30) Priorität: 19.05.2009 DE 202009007205 U
(43) Veröffentlichungstag der Anmeldung: 31.08.2011
(73) Patentinhaber: Plum A/S, 5610 Assens (DK)
(72) Erfinder: BERTELSEN, Poul, DK-5610 Assens (DK)
(74) Vertreter: Richter Werdermann Gerbaulet Hofmann
(86) Internationale Anmeldenummer: PCT/EP2010/003054
(87) Internationale Veröffentlichungsnummer: WO 2010/133350

(56) Entgegenhaltungen:
- US-A- 5 064 420
- US-A- 5 607 410
- US-A- 5 762 606
- US-A- 5 795 342

## Beschreibung

### Technisches Gebiet

Die Erfindung betrifft eine Augenspülvorrichtung nach dem Oberbegriff des Anspruches 1.

Augenspülvorrichtungen dienen zum Ausspülen eines oder beider Augen, die in Kontakt mit Chemikalien oder Staub bzw. Schmutz in Berührung gekommen sind.

### Stand der Technik

Aus der Patentschrift DE 26 39 449 C3 ist ein transportierbares Augenwaschgerät für industrielle Einsätze bekannt. Dieses umfasst ein Gehäuse, dass eine Ausnehmung hat, die so dimensioniert ist, dass sie den Kopf einer das Gerät benutzenden Person aufnehmen kann. Das Innere des Gehäuses ist hohl ausgebildet und bildet einen Behälter, in dem Waschflüssigkeit aufbewahrt wird. Ein Paar Düsen sind im Gehäuse an einander gegenüberliegenden Seiten vorgesehen. Jede Düse ist leicht nach oben gestellt, wodurch die Augen direkt besprüht und gewaschen werden können.

Eine weitere Augendusche ist aus der DE 20 2006 008 877 U1 bekannt. Diese umfasst eine Einspritzeinrichtung mit einem Anbringelement, der an handelsübliche Behälter für alkoholfreie Getränke befestigbar ist. Diese Augendusche besteht aus einem einteiligen Formstück aus einem Kunststoff. Dieses Formstück weist eine Augenmuschel mit einem elliptischen, umlaufenden Rand auf, mit dem die Augendusche an eine Flasche verschraubt werden kann.

Eine andere Lösung mit einem Schraubteil bzw. einem Flaschenaufsatz und einer Augenmuschel bzw. einem Augenspültrichter zeigt und beschreibt die DE 20 2007 002 927 U1. Hierbei soll ein ungewolltes Austreten der Flüssigkeit im Flaschenbereich verhindert werden. Aus einer oder mehreren Austrittsöffnungen des Trichters kann eine Spülflüssigkeit austreten. Zwischen dem Flaschenhals der Flasche und dem Flaschenaufsatz der Augendusche ist eine zusätzliche Dichtungseinrichtung vorgesehen.

Eine andere Augenspülflasche wird in der DE 33 02 868 A1 vorgestellt. Diese hat eine Augenmuschel, die mit einem Steigrohr verbunden ist. Das Steigrohr ist durch eine Überwurfmutter verschiebbar ausgeführt und trägt am Ende ein Sicherungselement, das ein Durchrutschen des Steigrohrs verhindert. Eine vergleichbare Konstruktion mit einem Steigrohr beschreibt die DE 83 02 343 U1.

Aus der DE 100 39 446 A1 ist eine andere Augenspülvorrichtung bekannt, die mit einem Spülflüssigkeit enthaltenen Behälter zu einem Augenspülgerät vereinigt werden kann. Diese Augenspülvorrichtung hat ein Augenkörbchen, das einen zum Aufsetzen auf das zu spülende Auge ausgebildeten Ring aufweist, der von vier Armen gehalten ist. Die Vorrichtung umfasst eine unterhalb des Ringes liegende Austrittsöffnung für die Flüssigkeit und ein die Austrittsöffnung speisendes Rohr.

Die Augenspülflasche, die in der DE 38 00 499 A1 beschrieben ist, zeichnet sich ebenfalls durch eine an einem Kopfstück angeordnete trichterförmige Augenmuschel aus. Weiterhin umfasst diese Spüleinrichtung ein Förderrohr, das mit einem Kopfstück verbunden ist und in eine Flasche eintauscht sowie eine brausenartige Austrittsöffnung, durch die die Flüssigkeit des Förderrohres geleitet wird, und ein Austrittsschlauch, an dem die herausgespritzte Flüssigkeit abgeleitet werden kann.

Ein Augenbecher hat den Nachteil, dass Verunreinigungen im Bereich des Augenbechers in das Auge des Benutzers gelangen können. Eine Augenspülvorrichtung zur Vermeidung dieses Problems ist aus der DE 601 25 796 T2 oder der DE 694 13 673 T2 bekannt. Die Augenspülvorrichtung hat eine Flasche, die eine Augenflüssigkeit enthält und mit einem Verschlusselement abgedichtet ist. Die Vorrichtung hat eine Hülse, die ein Augenbecher umgibt und dadurch diesen gegen Verschmutzung durch Staub und Schmutz schützt. Hier wird ebenfalls eine Augenwanne bestehend aus einer Schale eingesetzt, in der wenigstens eine Spritzdüse integriert ist. Verwendet wird ein Behälter für eine unter Druck stehende Flüssigkeit mit einem Austrittsventil sowie ein Regelorgan zum Regeln des an dem Ventil austretenden Flüssigkeitsstrahls.

Eine andere Augenspülvorrichtung ist aus der DE 882 470 B bekannt. Zum Spülen der Augen werden ein oder zwei Flüssigkeitsstrahlen verwendet, die von einer unter

Druck stehenden Flüssigkeitsquelle kommen und im spitzen Winkel nach oben einer sich nach den Flüssigkeitsstrahlen zu beugenden Person zugerichtet sind, wobei die betreffende Person mit den Händen die Augen auseinander spreizt. Die Vorrichtung besteht aus einer Schale, über die ein Augenspüler angeordnet ist.

Die in der DE 911 654 B beschriebene Augenspülvorrichtung hat einen Vorratsbehälter für eine neutralisierende Flüssigkeit mit einem Verbindungsschlauch, der mit einer um eine Achse drehbaren Brause verbunden ist. Dadurch sind eine hochgehobene Brausenstellung und eine Betriebsstellung möglich.

Ein Augentrichter einer auf einer Flasche aufschraubbaren Spülvorrichtung, der während des Spülvorgangs das Auge geöffnet halten soll, ist in der WO 87/02237 A1 beschrieben.

In der Druckschrift US 5 762 606 A wird eine Augenspülvorrichtung vorgestellt, die zum Halten der Augenlider einen oberen und einen unteren Halteflügel nutzt.

Die US 5 607 410 A beschreibt eine Augenspülvorrichtung einer anderen Art. Es wird nur ein Flügel eingesetzt, der durch Vakuum das obere Lid festhält. Zwei Leitungen werden zum Spülen verwendet. Durch eine Düse der ersten Leitung wird Flüssigkeit gesprüht, die von der zweiten Leitung bzw. einem Saugrohr nach der Augenspülung aufgesaugt wird.

Die US 5 064 420 A beschreibt eine Augenspülvorrichtung der gattungsgemäßen Art. Es sind als Flügel plattenartige Elemente mit einem gebogenen Querschnitt direkt mit einem Verbindungsteil einstückig verbunden. Insgesamt sind nur zwei Flügel vorhanden. Im Bereich einer Achse haben die Flügel eine geschlossene Fläche.

### Darstellung der Erfindung: Aufgabe, Lösung, Vorteile

Der Erfindung liegt die Aufgabe zugrunde, eine Augenspülvorrichtung zu schaffen, mit der das Auswaschen eines Auges in verbesserter Weise erfolgt.

Diese Aufgabe wird anspruchsgemäß dadurch gelöst, dass jeder Aufsatz (11) mit zwei Flügelpaaren (30, 31) ausgestattet ist, so dass vier Flügel vorhanden sind, wobei jedes Flügelpaar (30, 31) aus einem ersten bzw. oberen Flügel und einem zweiten bzw. unteren Flügel besteht, wobei die Flügelpaare (30, 31) voneinander beabstandet angeordnet sind, so dass eine Lücke zwischen den Flügelpaaren (30, 31) entsteht.

Die Erfindung schafft eine Erste-Hilfe-Vorrichtung zum Waschen bzw. Spülen eines Auges z.B. nach Anschluss an das Verspritzen einer Chemikalie, wie Säure, Lauge, Lösungsmittel, Giftstoffe und dergleichen.

Der Erfindung liegt der Gedanke zugrunde, dass beim Sprühen einer Auswasch-Flüssigkeit in das Auge dieser reflexartig geschlossen wird. Daher ist es bei bekannten Lösungen oft erforderlich, dass die Augenlider mit der Hand geöffnet werden müssen. Dies hat den Nachteil, dass eine Hand zum Spreizen der Augenlider und nicht zum Bedienen der Augenspülvorrichtung, z.B. zum Drücken einer Flasche mit Spülflüssigkeit, genutzt werden kann. Andere bekannte Lösungen eignen sich nur bedingt zum Spreizen der Augenlider.

Die erfindungsgemäßen Flügel wirken wie vier künstliche Hilfsfinger, die beide Augenlider auseinanderhalten, so dass die Spülflüssigkeit ungehindert gefährdete Augenbereiche treffen kann.

Ein Öffnen der Augenlider mit der Hand ist bei Augentrichtem nach dem Stand der Technik zum Teil nicht möglich, weil der Trichter eine geschlossene Wand um das Auge bildet, so dass kein Finger die Augenlider berühren kann.

Ein weiterer Vorteil der Erfindung ist, dass keine Augenmuschel vorhanden ist, die durch unsachgemäße Lagerung nicht steril sein kann. Spülflüssigkeit kann bei Augenmuscheln nicht optimal ablaufen, so dass an der Augenmuschel gelagerter Schmutz in das Auge gelangen kann. Dagegen kann bei der erfindungsgemäßen Lösung die Flüssigkeit optimal ablaufen.

Eine kompakte und handliche Ausführungsform der Augenspülvorrichtung ergibt sich dadurch, dass der Aufsatz direkt auf einen Behälter aufsetzbar ist, der als Flasche ausgeführt ist und die zusammendrückbar ist. Durch das Zusammendrücken der Flasche kann die Sprühflüssigkeit von unten nach oben bzw. positionsunabhängig und unabhängig von der Schwerkraft gesprüht werden.

Grundsätzlich reicht ein einziges Flügelpaar aus, um eine Öffnungsbewegung des Auges zu erreichen. Das Flügelpaar besteht erfindungsgemäß aus einem ersten bzw. oberen Flügel und einem zweiten bzw. unteren Flügel, so dass jeder Aufsatz mit zwei Flügelpaaren ausgestattet ist. Dies hat die Wirkung, als würde man das Auge mit vier Fingern bzw. zwei Händen öffnen, was wesentlich sicherer ist.

Weitere vorteilhafte Ausgestaltungen der Erfindung sind in den Unteransprüchen gekennzeichnet.

In einer vorteilhaften Weiterbildung der erfindungsgemäßen Vorrichtung ist vorgesehen, dass wenigstens eine Spülöffnung so gerichtet ist, dass sie direkt auf den Tränenkanal und/oder dessen Umgebung zielt. Jede Vorrichtung weist zum Beispiel ein Aufsatzteil mit einer kleinen, engen Öffnung auf, die in ihrer Anordnung und in ihrer Ausfluss- oder Ausspritzrichtung so ausgebildet ist, dass sie direkt auf den Tränenkanal bzw. dessen Umgebung zielt.

Bei einer weiteren vorteilhaften Ausführung der Erfindung ist wenigstens eine Spülöffnung so gerichtet, dass sie direkt auf die Pupille und/oder deren Umgebung zielt. Jedes Aufsatzteil weist zum Beispiel eine oder mehrere Öffnungen auf, die in ihrer Ausbildung und in ihrer Ausspritzrichtung so angeordnet und ausgebildet sind, dass die Spülflüssigkeit auf die Pupille bzw. dessen Umgebung auftrifft.

Eine einfache Befestigung an der Flasche ist dadurch möglich, dass der Aufsatz mit der Flasche verschraubbar ist.

Bei einer weiteren vorteilhaften Ausführung der Erfindung sind die flügelartigen Spreizelemente elastisch verformbar bzw. sind aus einem flexiblen Material. Beim Anpressen der Spreizelemente auf das Auge können sich diese Flügel weiter öffnen, wodurch ein Öffnen des Auges unterstützt wird. Optimal ist es hierfür, wenn die flügelartigen Spreizelemente eine bogenförmige Ausgestaltung aufweisen. Der dadurch gebildete Bogen öffnet sich beim Andrücken auf das Auge und entspannt sich, wenn es vom Auge entfernt wird.

Diese Öffnungsbewegung wird wesentlich weicher, wenn dazu noch die flügelartigen Spreizelemente mit wenigstens einer quer gerichteten Nut versehen sind, um eine Art Scharniergelenk zu bilden, um die die offenen Flügelenden schwenkbar sind. Die durch die Nut gebildete Materialschwachstelle ermöglicht ein Verbiegen des Flügels mit einer geringeren Kraft. Die Nut kann einseitig oder zweiseitig angebracht werden, um das Scharniergelenk auszubilden.

In einer vorteilhaften Weiterbildung des erfindungsgemäßen Antriebes ist vorgesehen, dass die flügelartigen Spreizelemente an den offenen Enden so abgeflacht bzw. gebogen sind, dass sie in etwa parallelen Kontakt zu dem Augenlid stehen. Die Endabschnitte der Flügel bilden jeweils eine Zunge, die an dem Augenlid, d.h. an dem oberen oder dem unteren Lid flach anliegt.

Damit die Flügel während des Anpressens nicht wegrutschen, ist es zweckmäßig, wenn an zu den Augenlidern gerichteten Kontaktseiten der Flügelenden eine rutschhemmende Oberflächenstruktur oder Profilierung angeordnet ist. Es kann zum Beispiel eine gummiartige Oberfläche als rutschhemmende Oberfläche oder es können zum Beispiel herausgeformte kleine Stege, Noppen der dergleichen an dem Flügelmaterial angeformt sein.

### Kurze Beschreibung der Zeichnungen

Ein Ausführungsbeispiel wird anhand der Zeichnungen näher erläutert, wobei weitere vorteilhafte Weiterbildungen der Erfindung und Vorteile derselben beschrieben sind.

Es zeigen:
Fig. 1 eine Darstellung einer auf ein Auge aufgesetzten erfindungsgemäßen Vorrichtung,
Fig. 2 eine perspektivische Darstellung eines Aufsatzes der Vorrichtung, und
Fig. 3 eine weitere Darstellung des Aufsatzes.

### Bester Weg zur Ausführung der Erfindung

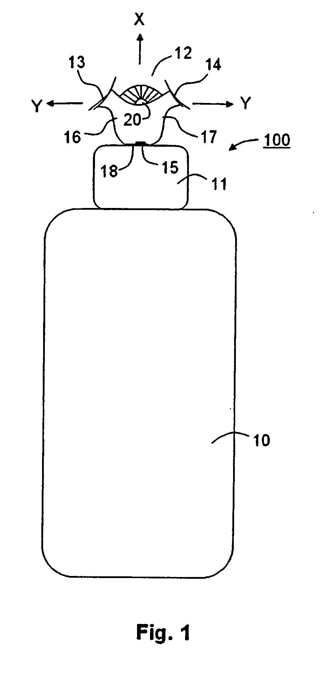

Die in Fig. 1 gezeigte Augenspülvorrichtung 100 dient zum Ausspülen eines Auges, das zum Beispiel mit einer Chemikalie, wie eine Säure, in Berührung gekommen ist.

Die Vorrichtung 100 ist mit einem Behälter 10 als verbindbaren, aufsetzbaren Aufsatz 11 ausgeführt. In dem Behälter 10 ist eine geeignete Spülflüssigkeit z.B. eine keimfreie Lösung enthalten. Wie Fig. 1 veranschaulicht, kann der Aufsatz 11 auf das Auge 12, insbesondere auf die Augenlider 13, 14 einer Person aufgesetzt werden.

Für den Austritt der Spülflüssigkeit ist der Aufsatz 11 mit wenigstens einer auf das Auge gerichteten Spülöffnung 15 versehen.

Erfindungsgemäß weist der Aufsatz 11 eine Einrichtung mit flügelartigen Spreizelementen 16, 17 auf, die das obere Augenlid 13 und das untere Augenlid 14 auseinanderziehen, um die Öffnung des Auges während des Spülvorgangs zu unterstützen. Dadurch ist es nicht erforderlich, die Augenlider mit zwei Fingern zu öffnen.

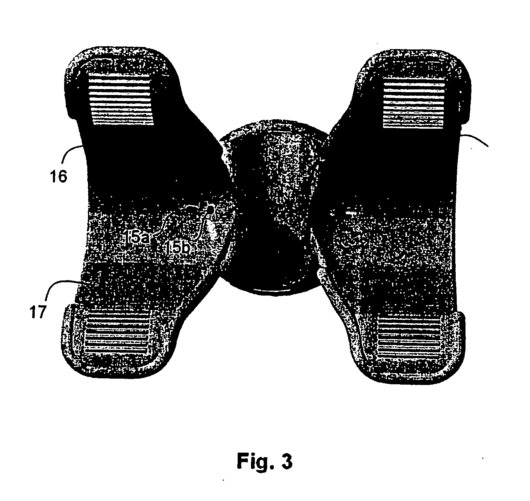

In einem Austrittsbereich 18 können eine oder mehrere Öffnungen vorhanden sein. Vorzugsweise ist wenigstens eine Spülöffnung 15a (Fig. 3) so gerichtet, dass sie direkt auf den Tränenkanal und/oder dessen Umgebung zielt. Alternativ oder zusätzlich ist wenigstens eine andere Spülöffnung 15b (Fig. 3) so gerichtet, dass sie direkt auf die Pupille 20 und/oder deren Umgebung zielt.

Wie Fig. 1 zeigt, ist der Aufsatz direkt auf einen Behälter 10 aufsetzbar, der als Flasche ausgeführt ist. Die Flasche kann aus einem flexiblen Kunststoff sein und ist zusammendrückbar, so dass ein ausreichender Spüldruck entsteht.

Der Aufsatz 11 hat ein Gewinde, so dass er mit der Flasche verschraubbar ist.

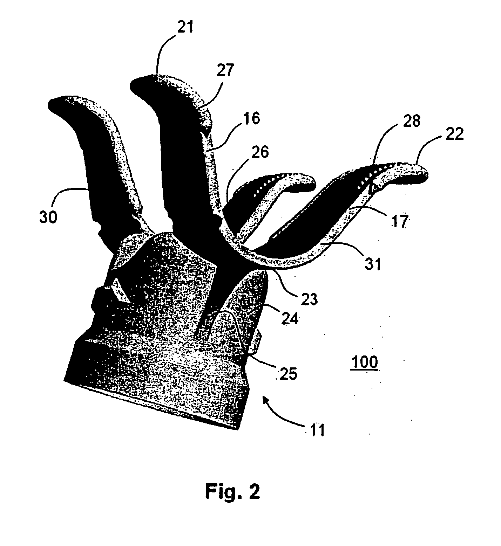

Die flügelartigen Spreizelemente 16, 17 sind elastisch verformbar. Durch Drücken der Flasche in Richtung X des Auges bewegen sich daher die Flügelenden 21, 22, die näher in Fig. 2 abgebildet sind, automatisch in Pfeilrichtung Y bzw. in entgegengesetzter Richtung, wie die Pfeile Y zeigen.

Die flügelartigen Spreizelemente 16, 17 weisen eine bogenförmige Ausgestaltung auf. Der Bogengrund 23 ist mit einem kappenförmigen Teil 24 des Aufsatzes 11 einstückig verbunden, und zwar so, dass er seitlich übersteht. Ein Versteifungssteg 25 verbindet zusätzlich das Spreizelement 16 mit dem kappenförmigen Teil 24.

Jedes der flügelartigen Spreizelemente 16, 17 ist mit wenigstens einer quer gerichteten Nut 26 versehen, um eine Art Scharniergelenk zu bilden, um die das offene Flügelende 21, 22 schwenkbar ist. Die Nut liegt zwischen dem Bogengrund 23 und dem Flügelende, und zwar etwa noch im unteren Bereich des Flügels bzw. auf etwa 1/3 der Strecke bis zum Flügelende 21.

Die Flügelenden sind randseitig mit einer Verdickung 27 versehen, die eine weiche Kante bildet, so dass das Auge nicht verletzt werden kann.

Wie die Figuren 1 und 2 zeigen, sind die flügelartigen Spreizelemente 16,17 an den offenen Enden 21, 22 so abgeflacht bzw. gebogen, dass sie in etwa parallelen Kontakt zu den Augenlidern 13,14 stehen. Dadurch verteilt sich der Druck in Richtung X auf einer größeren Fläche, was für den Benutzer angenehmer ist.

Fig. 2 zeigt, dass an zu den Augenlidern 13, 14 gerichteten Kontaktseiten der Flügelenden 21, 22 eine rutschhemmende Oberflächenstruktur oder Profilierung 28 angeordnet ist. Diese wird von kleinen, im Profil dreieckförmigen Stegen gebildet.

Insgesamt hat der Aufsatz 11 zwei Flügelpaare 30, 31, so dass vier Flügel vorhanden sind. Die Flügelpaare 30, 31 sind voneinander beabstandet angeordnet, so dass etwa eine Lücke zum Beispiel der Breite eines Flügels entsteht. Durch diese Lücke kann die Spülflüssigkeit optimal ablaufen, ohne dass sie eines der Flügel berühren muss, was von Vorteil ist, wenn die Flügel verschmutzt sind.

Wie Fig. 3 zeigt, verjüngen sich die Spreizelemente 16,17 zu ihren Enden 21, 22 hin.

Die Erfindung ist nicht auf dieses Beispiel beschränkt, so kann der Spüldruck auch mit Schwerkraft oder elektromotorisch entstehen. Auch kann die Augenspülvorrichtung auch zum Spülen des Auges mit einer medizinischen Lösung oder einer anderen Flüssigkeit vorgesehen sein, zum Beispiel bei Bindehautentzündungen. Anstelle eines Spülstrahls können auch Tropfen auf das Auge fallen, zum Beispiel medizinische Augentropfen. Die Begriffe Ausspülen, Spülöffnung und Spülvorgang können auch auf Tropfvorgänge angewendet werden, so dass die erfindungsgemäße Vorrichtung auch äquivalent auf eine Augentropfvorrichtung anwendbar ist. Auch kann die Vorrichtung für ein oder für beide Augen ausgeführt sein.

### Bezugszeichenliste

- 10: Behälter
- 11: Aufsatz
- 12: Auge
- 13, 14: Augenlider
- 15: Spülöffnungen
- 16, 17: Spreizelemente
- 18: Austrittsbereich
- 19: -
- 20: Pupille
- 21, 22: Flügelenden
- 23: Bogengrund
- 24: Kappenförmiges Teil
- 25: Versteifungssteg
- 26: Nut
- 27: Verdickung
- 28: Profilierung
- 29: -
- 30, 31: Flügelpaare

- 100: Augenspülvorrichtung

## Patentansprüche

1. Augenspülvorrichtung (100) zum Ausspülen wenigstens eines Auges (12), die mit einem Behälter (10) als verbindbaren aufsetzbaren Aufsatz (11) ausgeführt ist, wobei der Aufsatz (11) auf das Auge (12) einer Person aufgesetzt werden kann, und mit wenigstens einer auf das Auge (12) gerichteten Spülöffnung (15) versehen ist, wobei der Aufsatz (11) direkt dem Behälter (10) aufsetzbar ist, der als Flasche ausgeführt und zusammendrückbar ist, wobei der Aufsatz (11) eine Einrichtung mit flügelartigen Spreizelementen (16, 17) aufweist, die das obere Augenlid (13) und das untere Augenlid (14) auseinanderziehen, um die Öffnung des Auges (12) während des Spülvorgangs zu unterstützen, **dadurch gekennzeichnet, dass** jeder Aufsatz (11) mit zwei Flügelpaaren (30, 31) ausgestattet ist, so dass vier Flügel vorhanden sind, wobei jedes Flügelpaar (30, 31) aus einem ersten bzw. oberen Flügel und einem zweiten bzw. unteren Flügel besteht, wobei die Flügelpaare (30, 31) voneinander beabstandet angeordnet sind, so dass eine Lücke zwischen den Flügelpaaren (30, 31) entsteht.

2. Augenspülvorrichtung nach Anspruch 1, **dadurch gekennzeichnet, dass** wenigstens eine Spülöffnung (15a) so gerichtet ist, dass sie direkt auf den Tränenkanal und/oder dessen Umgebung zielt.

3. Augenspülvorrichtung nach Anspruch 1 oder 2, **dadurch gekennzeichnet, dass** wenigstens eine Spülöffnung (15b) so gerichtet ist, dass sie direkt auf die Pupille (20) und/oder deren Umgebung zielt.

4. Augenspülvorrichtung nach Anspruch 3, **dadurch gekennzeichnet, dass** der Aufsatz (11) mit der Flasche verschraubbar ist.

5. Augenspülvorrichtung nach einem der vorhergehenden Ansprüche, **dadurch gekennzeichnet, dass** die flügelartigen Spreizelemente (16, 17) elastisch verformbar sind.

6. Augenspülvorrichtung nach einem der vorhergehenden Ansprüche, **dadurch gekennzeichnet, dass** die flügelartigen Spreizelemente (16, 17) eine bogenförmige Ausgestaltung aufweisen.

7. Augenspülvorrichtung nach einem der Ansprüche 5 oder 6, **dadurch gekennzeichnet, dass** die flügelartigen Spreizelemente (16, 17) mit wenigstens einer quer gerichteten Nut (26) versehen sind, um eine Art Scharniergelenk zu bilden, um die die offenen Flügelenden (21, 22) schwenkbar sind.

8. Augenspülvorrichtung nach einem der vorhergehenden Ansprüche, **dadurch gekennzeichnet, dass** die flügelartigen Spreizelemente (16, 17) an den offenen Enden (21, 22) so abgeflacht bzw. gebogen sind, dass sie in etwa parallelen Kontakt zu dem Augenlid stehen.

9. Augenspülvorrichtung nach Anspruch 8, **dadurch gekennzeichnet, dass** an zu den Augenlidern (13, 14) gerichteten Kontaktseiten der Flügelenden (21, 22) eine rutschhemmende Oberflächenstruktur oder Profilierung (28) angeordnet ist.

## Claims

1. Eye-rinsing device (100) for rinsing at least one eye (12), which is designed with a container (10) as a connectably mountable attachment (11), wherein the attachment (11) can be applied to the eye (12) of a person and is provided with at least one rinsing opening (15) directed at the eye (12), whereby the attachment (11) can be directly mounted on the container (10), which is designed in the form of a bottle and can be compressed, whereby the attachment (11) comprises a device with wing-like spreading elements (16, 17) which pull apart the upper eyelid (13) and the lower eyelid (14) in order to support opening of the eye (12) during the rinsing procedure, **characterized in that** each attachment (11) is provided with two wing pairs (30, 31) so that four wings are available, whereby each wing pair (30, 31) consists of a first, or upper, wing and a second, or lower, wing, the wing pairs (30, 31) being arranged at a distance from one another so that a gap is produced between the wing pairs (30, 31).

2. Eye rinsing device according to claim 1 **characterized in that** at least one rinsing opening (15a) is aligned in such a way that it is directed directly at the tear duct and/or its surrounding area.

3. Eye rinsing device according to claim 1 or 2 **characterized in that** at least one rinsing opening (15b) is aligned in such a way that is directed directly at the pupil (20) and/or its surrounding area.

4. Eye rinsing device according to claim 3 **characterized in that** the attachment (11) with the bottle can be screwed on.

5. Eye rinsing device according to any one of the preceding claims **characterized in that** the wing-like spreading elements (16, 17) are elastically deformable.

6. Eye rinsing device according to any one of the preceding claims **characterized in that** the wing-lie spreading elements (16, 17) are arc-shaped.

7. Eye rising device according to any one of claims 5 or 6 **characterized in that** the wing-like spreading elements (16, 17) are provided with at least one transversely directed groove (26) in order to form a type of hinged joint about which the open wing ends (21, 22) can be pivoted.

8. Eye rinsing device in accordance with any one of the preceding claims **characterized in that** the wing-like spreading elements (16, 17) are flattened and/or curved at their open ends (21, 22) so that they are in approximately parallel contact with the eyelid.

9. Eye rinsing device according to claim 8 **characterized in** an anti-slip surface structure or profiling (28) is arranged on the contact sides of wing ends (21, 22) facing the eyelids (13, 14).

## Revendications

1. Dispositif de lavage des yeux (100) pour rincer au moins un oeil (12), qui est réalisé avec un récipient (10) en tant que calotte (11) à poser et à relier, la calotte (11) pouvant être posée sur l'oeil (12) d'une personne et qui est muni d'au moins un orifice de lavage (15) dirigé vers l'oeil (12), la calotte (11) pouvant se poser directement sur le récipient (10) qui est réalisé en tant que flacon et qui est compressible, la calotte (11) comportant un système avec des éléments d'écartement (16, 17) de type ailettes qui écartent la paupière supérieure (13) et la paupière inférieure (14) pour assister l'ouverture de l'oeil (12) pendant le processus de lavage, **caractérisé en ce que** chaque calotte (11) est équipée de deux paires d'ailettes (30, 31), de sorte que quatre ailettes soient présentes, chaque paire d'ailettes (30, 31) étant constituée d'une première ailette ou ailette supérieure et d'une deuxième ailette ou ailette inférieure, les paires d'ailettes (30, 31) étant disposées en étant écartées l'une de l'autre, de sorte à donner naissance à un intervalle entre les deux paires d'ailettes (30, 31).

2. Dispositif de lavage des yeux selon la revendication 1, **caractérisé en ce qu'**au moins un orifice de lavage (15a) est dirigé de sorte à viser directement le canal lacrymal et/ou son environnement.

3. Dispositif de lavage des yeux selon la revendication 1 ou la revendication 2, **caractérisé en ce qu'**au moins un orifice de lavage (15b) est dirigé de sorte à viser directement la pupille (20) et/ou son environnement.

4. Dispositif de lavage des yeux selon la revendication 3, **caractérisé en ce que** la calotte (11) peut se visser sur le flacon.

5. Dispositif de lavage des yeux selon l'une quelconque des revendications précédentes, **caractérisé en ce que** les éléments d'écartement (16, 17) de type ailettes sont élastiquement déformables.

6. Dispositif de lavage des yeux selon l'une quelconque des revendications précédentes, **caractérisé en ce que** les éléments d'écartement (16, 17) de type ailettes présentent une conception arquée.

7. Dispositif de lavage des yeux selon l'une quelconque des revendications 5 ou 6, **caractérisé en ce que** les éléments d'écartement (16, 17) de type ailettes sont munis d'au moins une rainure (26) dirigée à la transversale, pour former une sorte d'articulation en charnière, autour de laquelle les extrémités (21, 22) ouvertes des ailettes peuvent pivoter.

8. Dispositif de lavage des yeux selon l'une quelconque des revendications précédentes, **caractérisé en ce que** sur les extrémités ouvertes (21, 22), les éléments d'écartement (16, 17) de type ailettes sont aplatis ou recourbés, de sorte à être en contact approximativement parallèle avec la paupière.

9. Dispositif de lavage des yeux selon la revendication 8, **caractérisé en ce que** sur des faces de contact dirigées vers les paupières (13, 14) des extrémités d'ailettes (21, 22) est disposé(e) une structure superficielle antidérapante ou un profilage (28).
